(19) Europäisches Patentamt
European Patent Office
Office européen des brevets

(11) **EP 2 691 217 B1**

(12) **EUROPÄISCHE PATENTSCHRIFT**

(45) Veröffentlichungstag und Bekanntmachung des Hinweises auf die Patenterteilung:
**08.05.2019   Patentblatt 2019/19**

(21) Anmeldenummer: **12713044.1**

(22) Anmeldetag: **27.03.2012**

(51) Int Cl.:
*B26D 5/00* (2006.01)        *A22C 17/00* (2006.01)
*G01G 13/00* (2006.01)

(86) Internationale Anmeldenummer:
**PCT/EP2012/055431**

(87) Internationale Veröffentlichungsnummer:
**WO 2012/130853 (04.10.2012 Gazette 2012/40)**

(54) **VORRICHTUNG UND VERFAHREN ZUR AUTOMATISCHEN ÜBERWACHUNG EINER VORRICHTUNG ZUR VERARBEITUNG VON FLEISCHPRODUKTEN**

APPARATUS AND METHOD FOR AUTOMATICALLY MONITORING AN APPARATUS FOR PROCESSING MEAT PRODUCTS

DISPOSITIF ET PROCÉDÉ DE SURVEILLANCE AUTOMATIQUE D'UN DISPOSITIF DE TRAITEMENT DE PRODUITS CARNÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.03.2011   DE 102011015849**

(43) Veröffentlichungstag der Anmeldung:
**05.02.2014   Patentblatt 2014/06**

(73) Patentinhaber: **Nordischer Maschinenbau Rud. Baader GmbH + Co. KG**
**23560 Lübeck (DE)**

(72) Erfinder:
• **JÜRS, Michael**
  **23683 Haffkrug (DE)**
• **JACOBSEN, Ulf**
  **23611 Bad Schwartau (DE)**
• **PEDERSEN, Henning B.**
  **DK-7430 Ikast (DK)**

(74) Vertreter: **Stork Bamberger Patentanwälte PartmbB**
**Meiendorfer Strasse 89**
**22145 Hamburg (DE)**

(56) Entgegenhaltungen:
**US-B1- 7 251 537**

**Beschreibung**

Die Erfindung betrifft ein Verfahren zur automatischen Überwachung einer Vorrichtung zur Verarbeitung von Fleischprodukten, insbesondere Fisch.

Des Weiteren betrifft die Erfindung eine Überwachungsvorrichtung zur automatischen Überwachung einer Vorrichtung zur Verarbeitung von Fleischprodukten, insbesondere Fisch.

Solche Vorrichtungen und Verfahren kommen in unterschiedlichen Bereichen der Fleisch verarbeitenden Industrie, insbesondere der Fischverarbeitung oder Geflügelverarbeitung, zum Einsatz, in denen unverarbeitete oder auch teilweise vorverarbeitete Fleischprodukte automatisiert verarbeitet werden. Grundsätzlich können Fleischverarbeitungsvorrichtungen Fleischprodukt aus unterschiedlichen Kategorien, Formen oder Gewichtsbereichen verarbeiten. Für die unterschiedlichen noch zu verarbeitenden Fleischprodukte wird die Fleischverarbeitungsvorrichtung jeweils entsprechend angepasst. Dazu wird das noch zu verarbeitende Fleischprodukt vermessen, insbesondere dessen Höhe, Breite und/oder Länge. Entsprechend dieser Messergebnisse werden die Maschinenparameter der Fleischverarbeitungsvorrichtung neu eingestellt. So können beispielsweise bei Fischverarbeitungsvorrichtungen die Messerabstände von Bauchmessern, Flankenmessern oder Rückenmessern in Abhängigkeit der Rumpfhöhe des noch zu verarbeitenden Fisches eingestellt werden. Üblicherweise werden solche Einstellungen der Fleischverarbeitungsvorrichtung, wie beispielsweise die Messerabstände, Maschinenparameter genannt. Sind die Maschinenparameter auf das zu verarbeitende Fleischprodukt eingestellt, wird danach das Fleischprodukt durch die Fleischverarbeitungsvorrichtung verarbeitet. Nicht zuletzt für die Vermarktung des verarbeiteten Fleischproduktes werden diese nach der Verarbeitung gemessen. Bevorzugt werden hierbei die Länge, die Breite, die Höhe und/oder das Gewicht des Fleischproduktes gemessen.

Zum besseren Verständnis der Erfindung werden im Folgenden das einer Fleischverarbeitungsvorrichtung zugeführte Fleischprodukt als Eingangsfleischprodukt und das diesem entsprechende, von der Fleischbearbeitungsvorrichtung verarbeitete Fleischprodukt als Ausgangsfleischprodukt bezeichnet. Durch die Verarbeitung entstehen jedoch oftmals neben den Fleischprodukten, die ausgebeutet bzw. gewonnen werden sollen, auch weitere Fleischprodukte, die nicht ausgebeutet werden sollen. Im Weiteren soll der Anteil des Ausgabefleischproduktes, dessen Ziel es ist von der Fleischverarbeitungsvorrichtung ausgebeutet oder gewonnen zu werden, als Ausbeutefleischprodukt bezeichnet. Der restliche Anteil des Ausgabefleischproduktes soll als Trägerprodukt bezeichnet werden. Dabei muss das Trägerprodukt nicht zwingend als Träger für das Ausbeutefleischprodukt dienen. Vielmehr kann das Trägerprodukt auch Eingeweide oder andere Teile eines Tierkörpers umfassen.

Ein beispielhafter Zweck einer Fischverarbeitungsvorrichtung ist es, das Filetfleisch eines einer Fischverarbeitungsvorrichtung zugeführten Fischrumpfes von dem Grätengerüst zu trennen. Das Eingangsfleischprodukt wäre in diesem Fall der Fischrumpf. Das Ausgangsfleischprodukt wäre das ausgebeutete Filetfleisch sowie der restliche Teil des Fischrumpfes. Das Ausgangsprodukt unterteilt sich in das Ausbeutefleischprodukt, nämlich das Fischfilet, sowie in das Trägerprodukt, nämlich das restliche Ausgangsfleischprodukt, insbesondere das Grätengerüst.

Aus der DE 4204843 A1 sind ein Verfahren und eine Vorrichtung zum Bestimmen des Volumens, der Form oder des Gewichts von Fisch oder anderen Objekten bekannt. Danach wird zur Bestimmung des Volumens, der Form oder des Gewichts von Fischen von jedem Fisch, der zunächst auf einem Förderband transportiert wird, eine Bilderserie der Umrisse des Fisches mit einer Kamera aufgenommen. Anschließend wird das Volumen des Fisches oder sein Gewicht mit einem Mikroprozessor auf der Basis der empfangenen Bilddaten berechnet. Aus der Bilderserie der Kamera entsteht ein zusammengesetztes Bild des Fisches mit vielen Querschnitten, wobei die Breite und die maximale Dicke des jeweiligen Fisches in jedem Querschnitt gemessen werden. Das Volumen des Fisches wird durch Multiplizieren des Querschnittsbereiches mit der Geschwindigkeit des Förderbandes und der Zeit zwischen den einzelnen Bildern erhalten. Das Gewicht des Fisches wird dann durch Multiplizieren des Volumens mit dem spezifischen Gewicht der zu wiegenden Fischart erhalten.

Aus dem Stand der Technik ist es also bekannt, wie z.B. aus der US 7 251 537 B1, dass zu verarbeitende Fischprodukte vor ihrer Verarbeitung durch Sensoren vermessen werden, und dass aus diesen Sensordaten auf das Volumen oder das Gewicht des zu verarbeitenden Fisches geschlossen werden kann.

Außerdem ist bekannt, dass das Ausgabefleischprodukt vermessen wird, um so auf das Gewicht des Ausgabefleischproduktes zu schließen. Es kann jedoch keine Aussage darüber getroffen werden, ob das Ausbeuteprodukt demjenigen Anteil des Eingangsproduktes entspricht, der durch die Fleischbearbeitungsvorrichtung hätte gewonnen werden können oder ob und zu welchem Maß es zu Abweichungen gekommen ist. Vielmehr werden oft nur Messdaten des Eingangsproduktes, des Ausgangsproduktes und/oder des Ausbeuteproduktes erfasst.

Zur Verdeutlichung soll die Problemstellung an dem folgenden Beispiel erläutert werden: Durch eine Fleischverarbeitungsvorrichtung kann ein Filetfleisch (hier das gewünschte Ausbeutefleischprodukt) von den Knochen oder Gräten eines Eingangsfleischproduktes getrennt werden. Diese Trennung erfolgt jedoch nur zu einem gewissen Grad. Meistens bleiben noch Fleischreste an den Knochen oder Gräten hängen, so dass in den seltensten Fällen eine vollständige Trennung erfolgt. Die Ausbeute beschreibt somit denjenigen Fleischproduktanteil, der von dem Rest des zu verarbeitenden Eingangsfleischproduktes durch die Fleischverarbeitungsvorrichtung getrennt werden soll. Die absolute Ausbeute kann dann das Ausbeutefleischprodukt, hier also das abgetrennte Fleischprodukt, sein. Die relative Ausbeute kann das Verhältnis von dem Ausbeutefleischprodukt zu dem Anteil des Eingangsfleischproduktes sein, das hätte, insbesondere maximal, aus dem Eingangsfleischprodukt erbeutet oder gewonnen werden sollen.

Oftmals werden jedoch nur relative Ausbeuten von deutlich unter 100% erzielt. Denn aufgrund der vielfältigen Kategorien, Formen, Längen, Größen und/oder Höhen eines zu verarbeitenden Eingangsfleischproduktes werden besonders hohe Anforderungen an Fleischverarbeitungsvorrichtung gestellt. Nicht jede Fleischverarbeitungsvorrichtung ist gleichermaßen geeignet, um unterschiedliche Kategorien und/oder Formen von Fleischprodukten in der Weise zu verarbeiten, dass eine gleich hohe, insbesondere relative, Ausbeute erreicht wird. Vielmehr ist es nötig, dass die Maschinenparameter einer Fleischverarbeitungsvorrichtung auf das jeweils zu verarbeitende Fleischprodukt eingestellt werden. Dabei unterliegen die Maschinenparameter natürlichen Grenzen. So ist es üblich, dass Fischverarbeitungsvorrichtungen beispielsweise in der Weise ausgestaltet sind, dass sie nur Fische mit einer Rumpfhöhe aus einem vorbestimmten Rumpfhöhenbereich besonders gut im Sinne der Ausbeute verarbeiten können. Werden nun von dieser Fischverarbeitungsvorrichtung Fische mit der gleichen Rumpfhöhe verarbeitet, ist es dennoch möglich, dass jeweils eine unterschiedliche Ausbeute erzielt wird. Das ist beispielsweise darauf zurückzuführen, dass es schmale lange oder kurze breite oder auch mittellange und mittelbreite Fische mit der gleichen Rumpfhöhe gibt. Zwischen diesen Extremen sind eine Vielzahl von Formen denkbar und in der Praxis auch gegeben. Aufgrund der jeweils unterschiedlich ausgestalteten Form des zu verarbeitenden Fisches beziehungsweise Fleischproduktes ist es oftmals nicht möglich, dass die Fleischverarbeitungsvorrichtung das zu verarbeitende Fleischprodukt gleich gut in der Weise verarbeitet, dass jeweils die maximale Ausbeute erzielt wird. So können beispielsweise die Verarbeitungsmesser eine Fischverarbeitungsvorrichtung nicht jeder Kontur des Fischgrätenskelettes eines zu verarbeitenden Fisches folgen. Folglich bleibt immer noch ein Rest von Fleisch an den Gräten hängen. Dies führt zu einer absolut gesehenen schlechteren oder nicht optimalen Ausbeute.

Es ist also kein Fehler der Vorrichtung, wenn beispielsweise Fische mit der gleichen Rumpfhöhe von der gleichen Fleischverarbeitungsvorrichtung nur mit unterschiedlichen relativen und/oder absoluten Ausbeuten verarbeitet werden können. Allein aus der, insbesondere absoluten oder relativen, Ausbeute eines zu verarbeitenden Eingangsfleischproduktes ist deshalb keine Aussage über einen Fehler oder eine falsche Einstellung der Fleischverarbeitungsvorrichtung möglich.

Der Erfindung liegt die Aufgabe zugrunde, ein Verfahren und eine Vorrichtung zu schaffen, mit der die durch eine Fleischverarbeitungsvorrichtung erzielbare, insbesondere relative und/oder absolute, Ausbeute überwacht werden kann.

Die Aufgabe wird durch ein Verfahren mit den Merkmalen des Anspruchs 1 gelöst. Dabei werden zunächst Eingangssignale von Eingangssensoren gemessen. Bei den Eingangssensoren handelt es sich um Sensoren zur Erfassung von Geometrie- und/oder Gewichtsdaten des der Vorrichtung zur Fleischverarbeitung zugeführten Eingangsfleischproduktes. Als Eingangssignal kann das Signal verstanden werden, das von einem Eingangssensor ausgegeben und/oder verändert wird. So kann das Eingangssignal ein analoger Spannungsverlauf sein. Unter den Geometriedaten des Fleischproduktes können raumbezogene Daten des Fleischproduktes verstanden werden, wie die Länge, Breite, Höhe, Form und/oder Außenkontur des Fleischproduktes. Unter einem zugeführten Eingangsfleischprodukt kann sowohl ein vorverarbeitetes als auch unverarbeitetes Fleischprodukt verstanden werden. So kann ein zugeführtes Eingangsfleischprodukt ein unverarbeiteter Fisch oder auch ein bereits ausgenommener Fischrumpf sein.

Des Weiteren dient das Messen von Eingangssignalen von Eingangssensoren zur Ermittlung mindestens einer ausbeuterelevanten Prädiktionsgröße. Die Ermittlung kann auch eine bevorzugte Ausgestaltung der Erfindung sein. Bei der Prädiktionsgröße kann es sich eine die Form, das Gewicht und/oder das Volumen zumindest eines Abschnittes des zugeführten Eingangsfleischproduktes repräsentierenden ausbeuterelevanten Größe handeln. Wie bereits eingangs erläutert, handelt es sich bei der Ausbeute um den Fleischanteil, der durch die Fleischverarbeitungsvorrichtung von dem restlichen Fleischprodukt getrennt werden soll. Umfasst ein zugeführtes Eingangsfleischprodukt noch Knochen oder ein Knochengerüst beziehungsweise ein zugeführter Fisch noch Gräten oder ein Grätengerüst, so kann die Fleischbeziehungsweise Fischverarbeitungsvorrichtung in der Weise eingerichtet sein, um das eigentliche Fleisch von den Knochen, dem Knochengerüst, den Gräten oder dem Grätengerüst zu trennen. In der Praxis bleibt regelmäßig noch ein restlicher Teil des eigentlichen Fleisches an den Knochen, dem Knochengerüst, den Gräten oder dem Grätengerüst haften, so dass das eigentliche Fleisch nicht vollständig von dem restlichen Teil des Fleisches getrennt, erbeutet oder gewonnen werden kann. Ausbeuterelevant kann also der Fleischanteil des Fleischproduktes sein, der mittels der Fleischverarbeitungsvorrichtung von dem restlichen Teil des zugeführten Eingangsfleischproduktes getrennt werden soll. Mit anderen Worten kann unter einer ausbeuterelevanten Prädiktionsgröße eine Größe verstanden werden, mit der die ausbeutbare Form, das ausbeutbare Gewicht und/oder das ausbeutbare Volumen zumindest eines Abschnittes des zugeführten Eingangsfleischproduktes vorhergesagt werden kann. In einer besonders bevorzugten Ausgestaltung ist die ausbeuterelevante Prädiktionsgröße das erwartete ausbeutbare Gewicht, zumindest eines Abschnittes, des zugeführten Eingangsfleischproduktes oder Fisches. In einer besonders bevorzugten Ausgestaltung kann unter der aubeuterelevanten Prädiktionsgröße das erwartete Gewicht, die erwarteten geometrischen Abmessungen und/oder die erwartete Form des Ausbeuteprodukts verstanden werden. Es ist also bevorzugt, dass die ausbeutbare Prädiktionsgröße nicht das ganze Volumen und/oder das ganze Gewicht des zugeführten Eingangsfleischproduktes oder Fisches bestimmt, sondern nur dessen ausbeutbaren Teil.

Das erfindungsgemäße Verfahren ist des Weiteren gekennzeichnet durch ein Messen von Ausgangssignalen der Ausgangssensoren zur Erfassung von Geometrie- und/oder Gewichtsdaten eines, insbesondere eines entsprechenden, vorzugsweise dem Eingangsfleischprodukt entsprechenden, Fleischproduktes, vorzugsweise des Ausbeutefleischproduktes, und zur Ermittlung von einer ausbeuterelevanten Ertragsgröße. Dabei kann die Ermittlung auch eine vorteilhafte Ausgestaltung der Erfindung sein. Mit den Ausgangssensoren können also Geometriedaten und/oder Gewichtsdaten des Ausbeutefleischproduktes erfasst werden. Unter einem Ausgangssignal kann ein Signal verstanden werden, das von dem Ausgangssensor stammt oder von diesem verändert wurde, wie beispielsweise ein analoger Spannungsverlauf. Damit die Ausgangssensoren Daten des Ausbeutefleischproduktes erfassen können, können die Ausgangssensoren im Abtransportbereich der Fleischverarbeitungsvorrichtung angeordnet sein. Des Weiteren ist es möglich, dass die Ausgangssensoren hinter der Fleischverarbeitungsvorrichtung angeordnet sind. Mittels der Ausgangssignale der Ausgangssensoren kann mindestens eine ausbeuterelevante Ertragsgröße ermittelt werden. Unter der ausbeuterelevanten Ertragsgröße kann eine die ausbeuterelevante Form, das ausbeuterelevante Gewicht und/oder das ausbeuterelevante Volumen des Ausbeutefleischproduktes repräsentierenden Größe verstanden werden. In einer besonders bevorzugten Ausgestaltung ist die ausbeuterelevante Ertragsgröße das Gewicht des Ausbeutefleischproduktes.

Des Weiteren zeichnet sich das erfindungsgemäße Verfahren durch ein Berechnen einer Differenzgröße mittels einer Differenzeinheit durch Differenzbildung zwischen mindestens einer der Prädiktionsgrößen und der mindestens einen entsprechenden Ertragsgröße aus. Eine besonders bevorzugte Ausgestaltung zeichnet sich durch eine Differenzbildung zwischen einer der Prädiktionsgrößen und der einen entsprechenden Ertragsgröße aus. Ist das zu verarbeitende Fleischprodukt ein Fisch und wird der Rumpf des Fisches in zwei Abschnitte unterteilt, so kann für jeden Abschnitt des Fischrumpfes eine Prädiktionsgröße ermittelt werden. Werden des Weiteren durch eine Fischverarbeitungsvorrichtung die beiden Abschnitte des Rumpfes des Fisches von dem restlichen Fisch getrennt, können die Geometrie- und/oder Gewichtsdaten der verarbeitenden und abgetrennten Abschnitte des Fischrumpfes erfassen und daraus zwei ausbeuterelevante Ertragsgrößen ermitteln. Jedem Abschnitt des Fischrumpfes ist in dem genannten Beispiel jeweils ein Prädiktionswert und ein Ertragswert zugeordnet. Deshalb kann die Differenzbildung durch die Differenzeinheit zwischen einer dem einen der Abschnitte zugeordneten Prädiktionsgröße und der dem gleichen Abschnitt zugeordneten Ertragsgröße, also entsprechenden Ertragsgröße, erfolgen, indem zwischen der Prädiktionsgröße und der entsprechenden Ertragsgröße die Differenzgröße berechnet wird. Mit anderen Worten kann die Differenzgröße in der Weise berechnet werden, dass von der Prädiktionsgröße die Ertragsgröße abgezogen wird oder von der Ertragsgröße die Prädiktionsgröße abgezogen wird. Sofern für das zugeführte Eingangsfleischprodukt oder für einen Abschnitt des zugeführten Eingangsfleischproduktes mehrere ausbeuterelevante Prädiktionsgrößen ermittelt worden sind und/oder sofern für das Ausbeutefleischprodukt oder für einen Abschnitt des Ausbeutefleischproduktes mehrere ausbeuterelevante Ertragsgrößen ermittelt worden sind, kann die Differenzbildung auch zwischen einer der Prädiktionsgrößen und mehrerer entsprechenden Ertragsgrößen oder zwischen mehreren Prädiktionsgrößen und einer der Ertragsgrößen erfolgen. Sofern mehrere Prädiktionsgrößen ermittelt worden sind, können diese auch zu einer gemeinsamen Prädiktionsgröße addiert werden. Sofern mehrere ausbeuterelevante Ertragsgrößen ermittelt werden, können diese auch zu einer gemeinsamen ausbeuterelevanten Ertragsgröße addiert werden.

Eine vorteilhafte Weiterbildung der Erfindung sieht vor, dass die Prädiktionsgröße aus den Eingangssignalen mittels eines Morphologiemodells berechnet wird. Das Morphologiemodell kann ein spezielles Morphologiemodell für das zu verarbeitende Fleischprodukt, insbesondere Fisch, sein. Für die Verarbeitung von Fischen kann der Korpulenzfaktor (K-Faktor oder KF) in dem Morphologiemodell berücksichtigt werden. Der K-Faktor kann für jeden Fisch, beziehungsweise für jede Fischkategorie unterschiedlich sein. Des Weiteren kann ein Ausbeutefaktor (AF) in dem Morphologiemodell berücksichtigt werden, wobei der Ausbeutefaktor für jeden Fisch unterschiedlich sein kann. Ein einfaches Morphologiemodell für einen Fisch könnte unter Berücksichtigung der zuvor genannten Faktoren wie folgt ausgestaltet sein:

$$\textit{Ausbeuterelevante Prädiktionsgröße} = (\textit{Länge des Fisches})^3 \; x \; KF \; x \; AF : 100.$$

Grundsätzlich können der Korpulenzfaktor und/oder der Ausbeutefaktor von den gemessenen Geometrie- und/oder Gewichtsdaten abhängen.

Dadurch, dass es sich bei der Prädiktionsgröße und der Ertragsgröße jeweils um ausbeuterelevante Größen handelt, kann mittels der daraus resultierenden Differenzgröße die erzielbare Ausbeute überwacht werden. Denn es wird nicht das gesamte Volumen und/oder gesamte Gewicht des zugeführten Eingangsfleischproduktes mit dem des Ausgabe- oder Ausbeutefleischproduktes verglichen. Vielmehr erfolgt eine Differenzbildung zwischen den dafür relevanten Größen, nämlich zwischen den ausbeuterelevanten Größen. Mit anderen Worten kann die Fleischverarbeitungsvorrichtung entkoppelt oder unabhängig von den Anteilen des zugeführten Eingangsfleischproduktes überwacht werden, die nicht von der Fleischverarbeitungsvorrichtung ausgebeutet oder nicht von dem restlichen Teil des Fleischproduktes getrennt werden soll. Nochmal anderes ausgedrückt, ermöglicht die vorliegende Erfindung eine Überwachung der Fleischverarbeitungsvorrichtung mittels der Daten desjenigen Teils oder Abschnitts des zugeführten Eingangsfleischproduktes, welches mittels der Fleischverarbeitungsvorrichtung von dem restlichen Teil beziehungsweise Abschnitt des zugeführten Eingangsfleischproduktes abgetrennt werden soll. Somit kann eine objektive Überwachung der Fleischverarbeitungsvorrichtung anhand der ausbeuterelativen Teile beziehungsweise Abschnitte des zugeführten Eingangsfleischproduktes und des Ausbeutefleischproduktes erfolgen.

Neben einer erzielbaren Ausbeute kann aus der Differenzgröße auch eine Aussage über die Effizienz der Fleischverarbeitungsvorrichtung gewonnen werden. So kann die Effizienz der Fleischverarbeitungsvorrichtung als eine linear abhängige Größe von der Differenzgröße betrachtet werden. Die Effizienz kann in einer vorteilhaften Ausgestaltung der Erfindung auch optisch und/oder akustisch ausgegeben werden, insbesondere mittels einer Ausgabevorrichtung.

Des Weiteren kann es vorteilhaft sein, wenn nicht nur über die Effizienz der Vorrichtung zur Fleischverarbeitung eine Aussage getroffen werden kann, sondern auch darüber, ob die Fleischverarbeitungsvorrichtung das Fleischprodukt hätte besser verarbeiten können oder ob die Fleischverarbeitungsvorrichtung fehlerhaft eingestellt ist und/oder ob die Fleischverarbeitungsvorrichtung grundsätzlich fehlerhaft arbeitet.

Eine bevorzugte Ausgestaltung der Erfindung zeichnet sich dadurch aus, dass die Prädiktionsgröße aus den Eingangssignalen und Maschinenparametern mittels eines Morphologiemodells und eines Maschinenmodells berechnet wird. Dabei kann das Morphologiemodell wie zuvor erläutert ausgestaltet sein. Unter Maschinenparameter können grundsätzlich sämtliche Parameter zur Einstellung der Fleischverarbeitungsvorrichtung verstanden werden. Bei einer Fischverarbeitungsvorrichtung können dies beispielsweise die Parameter sein, die den Messerabstand der Bauchmesser, Flankenmesser und/oder Rückenmesser bestimmen. Wird beispielsweise durch die Eingangssensoren eines zu verarbeitenden Fisches der Außendurchmesser (DA) des Fisches ermittelt sowie durch ein Morphologiemodell der Außendurchmesser des Grätengerüsts (DI) ermittelt, und haben die Bauchmesser der Fischverarbeitungsvorrichtung einen bestimmten Messerabstand (MA) so kann ein einfaches Maschinemodell wie folgt ausgestaltet sein

$$MF = \left\{ \begin{array}{ll} \left( \dfrac{DA - MA}{DA - DI} \right) & \textit{für } DA > MA > DI \\ 0 & \textit{sonst} \end{array} \right\}$$

MF bezeichnet hierbei den Maschinenfaktor. Der Maschinenfaktor kann auch bei der Berücksichtigung der Prädiktionsgröße Einfluss finden. Die Prädiktionsgröße kann somit mittels des Morphologiemodells und des Maschinenmodells aus den Eingangssignalen wie folgt bestimmt werden:

$$\textit{Prädiktionsgröße} = (\textit{Länge des Fisches})^3 \; x \; KF \; x \; AF \; x \; MF : 100.$$

Durch die Eingangssignale beziehungsweise Geometrie- und/oder Gewichtsdaten des zugeführten Eingangsfleischproduktes und mittels eines Morphologie- und Maschinenmodells können auch diejenigen Informationen in die Ermittlung der Prädiktionsgröße eingehen, die die Anpassung der Fleischverarbeitungsvorrichtung auf das zu verarbeitende Fleischprodukt beschreiben.

Eine weitere vorteilhafte Ausgestaltung der Erfindung zeichnet sich dadurch aus, dass der Prädiktionsgröße in Abhängigkeit der Eingangssignale oder der Eingangssignale und Maschinenparameter ein entsprechender Wert aus einer Datenbank zugeordnet wird, wobei in der Datenbank Prädiktionsgrößen in Abhängigkeit von Eingangssignalen oder Eingangssignalen und Maschinenparametern gespeichert sind. In einer besonders einfachen Ausgestaltung weist die Datenbank eine Vielzahl von unterschiedlichen Kombinationen der Eingangssignale beziehungsweise Eingangssignale und Maschinenparameter auf, wobei für jede Kombination der Eingangssignale oder für jede Kombination der Eingangssignale und Maschinenparameter eine entsprechende Prädiktionsgröße in der Datenbank gespeichert ist. Werden Eingangssignale der Eingangssensoren zur Erfassung von Geometrie- und/oder Gewichtsdaten des der Vorrichtung zugeführten Eingangsfleischproduktes gemessen und werden vorzugsweise diejenigen Maschinenparameter berücksichtigt, die für das Fleischprodukt vorgesehen sind, um dies zu verarbeiten, kann ein Abgleich mit den Werten der Eingangssignale beziehungsweise Eingangssignale und Maschinenparameter aus der Datenbank in der Weise erfolgen, dass genau die gleichen Parameter in der Datenbank gefunden werden oder dass derjenige Datensatz der Eingangssignale beziehungsweise Eingangssignale und Maschinenparameter in der Datenbank gefunden wird, der, insbesondere im Mittel, die kleinste Abweichung aufweist. Der Prädiktionsgröße kann dann die Größe zugeordnet werden, die in der Datenbank entsprechend des ermittelten Datensatzes für die Prädiktionsgröße gespeichert ist. Mit anderen Worten können die Eingangssignale oder die Eingangssignale und die Maschinenparameter verwendet werden, um aus einer Datenbank eine den Eingangssignalen oder Eingangssignalen und Maschinenparametern entsprechenden Prädiktionsgröße auszulesen.

Eine weitere vorteilhafte Ausgestaltung der Erfindung zeichnet sich durch ein Ermitteln einer Vergleichsgröße durch Vergleichen der Differenzgröße mit einer Bezugsgröße aus. Die Bezugsgröße kann wahlweise vorbestimmt, berechnet oder zugeordnet sein. Unter dem Vergleichen kann eine Differenzbildung oder Verhältnisbildung verstanden werden. Ist die Bezugsgröße beispielsweise durch einen Wert von 10 vorbestimmt und wird aus der ausbeuterelevanten Prädiktionsgröße und der ausbeuterelevanten Ertragsgröße durch Differenzbildung eine Differenzgröße von beispielsweise 5 ermittelt, so kann die Differenzgröße mit der vorbestimmten Bezugsgröße verglichen werden. Bei der Differenzbildung wäre die Vergleichsgröße 5. Bei einer Verhältnisbildung der Differenzgröße zu der Bezugsgröße wäre die Vergleichsgröße 0,5. Durch das Vergleichen der Differenzgröße mit der Bezugsgröße kann der Aussagegehalt der Differenzgröße objektiviert werden. Die Bezugsgröße kann deshalb als ein Maß oder als ein Maßstab für die Differenzgröße betrachtet werden. Wird also die Vergleichsgröße durch Differenzbildung zwischen der Differenzgröße und der Bezugsgröße ermittelt, so kann eine Aussage in der Weise erfolgen, dass die Vorrichtung zur Fischverarbeitung eine hohe Ausbeute hat, wenn die Vergleichsgröße klein ist, eine mittlere Ausbeute hat, wenn die Vergleichsgröße mittelgroß ist und eine kleine Ausbeute hat, wenn die Vergleichsgröße groß ist. Dabei kann eine Vergleichsgröße als klein angesehen werden, wenn diese zwischen 0 und 5 ist, als mittel angesehen werden, wenn diese zwischen 5 und 10 ist und als groß angesehen werden, wenn diese größer als 10 ist. Diese Werte sind nur beispielhaft zu verstehen, denn je nach Gewicht, Volumen, Länge, Höhe und/oder Breite des Ausbeutefleischproduktes, kann die Differenzgröße stark variieren. Ist die Differenzgröße von beispielsweise 5 (Gramm) als besonders klein anzusehen für ein zu verarbeitendes Fleischprodukt mit einem Gesamtgewicht von 2 (Kilogramm), so kann die gleiche Differenzgröße als groß angesehen werden, wenn das zu verarbeitende Fleischprodukt beispielsweise ein Gesamtgewicht von 70 (Gramm) aufweist. Ferner kann die Differenzgröße auch davon abhängen, inwieweit sich die Verarbeitungsvorrichtung auf das zu verarbeitende Fleischprodukt einstellen lässt. Ist beispielsweise der minimale Messerabstand für die Bauchmesser bei einem zu verarbeitenden Fisch größer oder deutlich größer als der Außendurchmesser des Grätengerüstes im Bauchbereich, so wird die Fischverarbeitungsvorrichtung, auch wenn sie fehlerfrei eingestellt ist und/oder optimal arbeitet, nicht das gesamte Bauchfleisch von dem Grätengerüst trennen können.

Es stellt sich also auch hier die Frage, wie eine Überwachungseinrichtung ausgestaltet sein kann, um eine Aussage darüber treffen zu können, ob die Fleischverarbeitungsvorrichtung das Fleischprodukt besser verarbeiten könnte, ob die Fleischverarbeitungsvorrichtung fehlerhaft oder nicht optimal eingestellt ist oder ob die Fleischverarbeitungsvorrichtung grundsätzlich fehlerhaft arbeitet.

Eine vorteilhafte Ausgestaltung der Erfindung zeichnet sich durch ein Berechnen der Bezugsgröße aus den Eingangssignalen und den Maschinenparametern mittels des Maschinenmodells und Morphologiemodells aus. Unter Berücksichtigung des Morphologiemodells und des Maschinenmodells kann eine Aussage darüber getroffen werden, wie gut die Vorrichtung beziehungsweise die Maschineneinstellungen, insbesondere bestimmt durch die Maschinenparameter, auf den zu verarbeitenden Fisch passen. Durch die Eingangssignale und das Morphologiemodell kann auf die Struktur, den Aufbau, die ausbeuterelevante Prädiktionsgröße und weitere Geometrie- und/oder Gewichtsdaten des zu verarbeitenden Fisches geschlossen werden. Sind also die Eingangssignale und die Maschinenparameter sowie ein Morphologiemodell und ein Maschinenmodell bekannt, kann daraus berechnet werden, wie groß die Differenz zwischen der ausbeuterelevanten Prädiktionsgröße und der ausbeuterelevanten Ertragsgröße sein wird, wobei die errechnete Differenz als Bezugsgröße verstanden werden kann.

Eine weitere vorteilhafte Ausgestaltung der Erfindung zeichnet sich dadurch aus, dass der Bezugsgröße in Abhängigkeit der Eingangssignale und Maschinenparameter oder in Abhängigkeit der Prädiktionsgröße und Maschinenparameter ein entsprechender Wert aus einer, insbesondere weiteren, Datenbank zugeordnet wird, wobei in der Datenbank Bezugsgrößen in Abhängigkeit von Eingangssignalen und Maschinenparametern oder in Abhängigkeit von Prädiktionsgrößen und Maschinenparametern gespeichert sind. Bereits zuvor wurde das Verfahren einer Zuordnung eines Wertes aus einer Datenbank beschrieben. Dieses gilt analog auch für die zuletzt genannte Datenbank unter Berücksichtigung der für diese Datenbank relevanten Größen und Parameter.

Mit anderen Worten kann unter der Bezugsgröße eine erwartete Differenzgröße verstanden werden. Denn in die berechnete oder zugeordnete Bezugsgröße können die Informationen eingegangen sein, die zu einer bestimmten Differenzgröße führen. Wenn beispielsweise die Bauchmesser nicht das gesamte Bauchfleisch eines Fisches abtrennen können, weil die Fischverarbeitungsvorrichtung gar nicht dazu geeignet ist, ist dies kein Fehler der Fischverarbeitungsvorrichtung und auch keine fehlerhafte Einstellung der Fischverarbeitungsvorrichtung. Diese analogen und/oder weiteren Daten können zur Berechnung oder Zuordnung der Bezugsgröße berücksichtigt werden. Durch den Vergleich der Bezugsgröße mit der Differenzgröße ist eine objektive Aussage über den Zustand der Vorrichtung möglich.

Eine weitere vorteilhafte Ausgestaltung der Erfindung zeichnet sich durch ein Erzeugen eines Steuersignals aus, wenn die Differenzgröße eine Toleranzgrenze der Bezugsgröße erreicht, überschreitet oder unterschreitet. Dabei kann die Bezugsgröße einen Toleranzbereich aufweisen. Der Toleranzbereich kann durch eine obere Toleranzgrenze und/oder eine untere Toleranzgrenze begrenzt sein. Die untere Toleranzgrenze kann einen Wert aufweisen, der kleiner ist als die Bezugsgröße. Die obere Toleranzgrenze kann einen Wert aufweisen, der größer ist als die Bezugsgröße. Mit anderen Worten kann der Bezugsgröße Toleranzgrößen zugeordnet sein, die kleiner beziehungsweise größer als die Bezugsgröße sind. Durch den Toleranzbereich beziehungsweise die Toleranzgrenzen kann der Bereich um eine Bezugsgröße bestimmt sein, der für die Differenzgröße toleriert wird. Das Steuersignal kann ein analoges oder digitales Signal sein. Insbesondere kann es ein Spannungssprung in einem analogen Signal sein.

Das durch die Vorrichtung zur Fleischverarbeitung verarbeitete Fleischprodukt wird oftmals in unterschiedliche Abschnitte oder Teile geteilt. So kann beispielsweise das Fleisch eines Fischrumpfes in drei Abschnitte oder Teile unterteilt sein. Diese Teile des verarbeiteten Fisches werden dann bevorzugt separat und/oder nacheinander aus der Vorrichtung zur Fischverarbeitung abtransportiert. Insbesondere um eine Differenzgröße zwischen sich entsprechenden Prädiktionsgrößen und Ertragsgrößen zu ermitteln, kann es sinnvoll sein, entsprechende Abschnitte des zugeführten Eingangsfleischproduktes zu erkennen. Eine vorteilhafte Ausgestaltung der Erfindung zeichnet sich durch ein Identifizieren eines Abschnittes des zugeführten Eingangsfleischproduktes aus den Eingangssignalen mittels eines Morphologiemodells aus. Das Identifizieren kann auch oder alternativ mittels eines Maschinenmodells erfolgen. Dabei ist es vorteilhaft, wenn die geometrischen Grenzen des jeweiligen Abschnittes den geometrischen Auskanten des Ausbeutefleischproduktes entsprechen. Wird also beispielsweise ein Fleischprodukt in drei gleich breite Streifen getrennt, so können die Abschnitte des zugeführten Fleischprodukts in der Weise identifiziert werden, dass das zugeführte Eingangsfleischprodukt auch in drei gleich breite Abschnitte, nicht physisch aber insbesondere virtuell, scheinbar und/oder funktional unterteilt wird. Die auf diese Weise identifizierten Abschnitte des zugeführten Eingangsfleischproduktes entsprechen in ihrer geometrischen Form zumindest annähernd der geometrischen Form des Ausbeutefleischproduktes. Bis zu 1, 2, 3, 5, 7, 10, 12 oder 15% Längenunterschied zwischen den identifizierten Abschnitten und den verarbeiteten Abschnitten können akzeptabel sein. Werden die einzelnen Abschnitte des zugeführten Eingangsfleischproduktes nicht identifiziert, so kann es sinnvoll sein, dass eine Differenzbildung zwischen der Prädiktionsgröße des zugeführten Eingangsfleischproduktes und der Summe der entsprechenden Ertragsgrößen des Ausbeutefleischproduktes erfolgen.

Eine weitere vorteilhafte Ausgestaltung der Erfindung zeichnet sich dadurch aus, dass ein Abschnitt des zugeführten Eingangsfleischproduktes in Abhängigkeit der Eingangssignale mittels einer, insbesondere weiteren, Datenbank identifiziert wird. In dieser Datenbank können Abschnittsdaten von Fleischprodukten in Abhängigkeit von Eingangssignalen gespeichert sein. Bei den Abschnittsdaten kann es sich um Daten oder Werte handeln, die die entsprechende Geometrie und/oder Außenkontur repräsentieren, bestimmen oder bestimmbar machen. Mit anderen Worten kann es sich bei den Abschnittsdaten um einen Abschnitt repräsentierende Werte und/oder Daten handeln. Nach einer Ausgestaltung der Erfindung kann einem Abschnitt des zugeführten Eingangsfleischproduktes der Wert zugeordnet werden, der in der Datenbank zu dem entsprechenden gemessenen Eingangssignal gespeichert ist. Des Weiteren kann die Zuordnung eines Wertes aus einer Datenbank analog zu den bereits erläuterten Methoden erfolgen.

Die eingangs genannte Aufgabe wird auch durch eine Überwachungsvorrichtung zur automatischen Überwachung einer Vorrichtung zur Verarbeitung von Fleischprodukten, insbesondere Fisch, gelöst mit einer Prädiktionseinheit zur Ermittlung einer ausbeuterelevanten Prädiktionsgröße, wobei die Prädiktionseinheit mit Eingangssensoren zur Erfassung von Geometrie- und/oder Gewichtsdaten des der Vorrichtung zur Fleischverarbeitung zugeführten Eingangsfleischproduktes mittels einer Datenverbindung verbunden ist, einer Ertragsermittlungseinheit zur Ermittlung mindestens einer ausbeuterelevanter Ertragsgröße, wobei die Ertragsermittlungseinheit mit den Ausgangssensoren zur Erfassung von Geometrie- und/oder Gewichtsdaten des Ausbeutefleischproduktes mittels einer weiteren Datenverbindung verbunden ist, und einer Differenzeinheit zur Berechnung einer Differenzgröße aus der Differenz zwischen, insbesondere mindestens, einer der Prädiktionsgrößen und der mindestens einen entsprechenden Ertragsgröße, wobei die Differenzeinheit mit der Prädiktionseinheit durch eine weitere Datenverbindung verbunden ist, und wobei die Differenzeinheit mit der Ertragsermittlungseinheit durch eine weitere Datenverbindung verbunden ist.

Weitere zweckmäßige und/oder vorteilhafte Merkmale und/oder Weiterbildungen der Erfindung ergeben sich aus den Unteransprüchen und der Beschreibung. Eine besonders bevorzugte Ausgestaltung wird anhand der beigefügten Zeichnungen näher erläutert. In den Zeichnungen zeigen:

Fig. 1A     eine Darstellung eines Transportsattels mit einem Fischrumpf in perspektivischer Ansicht,

Fig. 1B     eine Darstellung eines Transportsattels mit einem Fischrumpf in Vorderansicht,

Fig. 1C     eine Darstellung eines Transportsattels mit Fischrumpf in geschnittener Seitenansicht,

Fig. 2 A    eine schematische Darstellung eines Blockschaltbildes einer Überwachungsvorrichtung zur automatischen Überwachung einer Fleischverarbeitungsvorrichtung, und

Fig. 2 B    eine schematische Darstellung eines Blockschaltbildes einer Überwachungsvorrichtung zur automatischen Überwachung einer Fleischverarbeitungsvorrichtung mit weiteren vorteilhaften Ausgestaltungen.

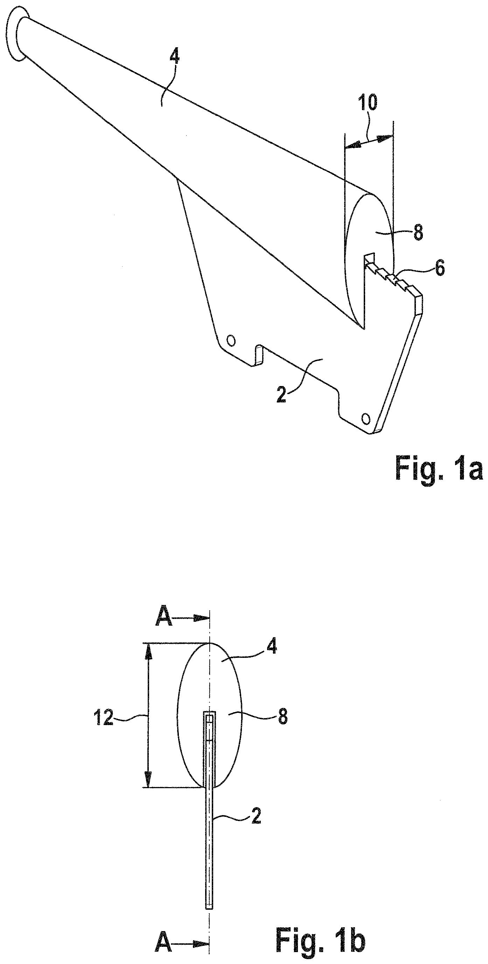

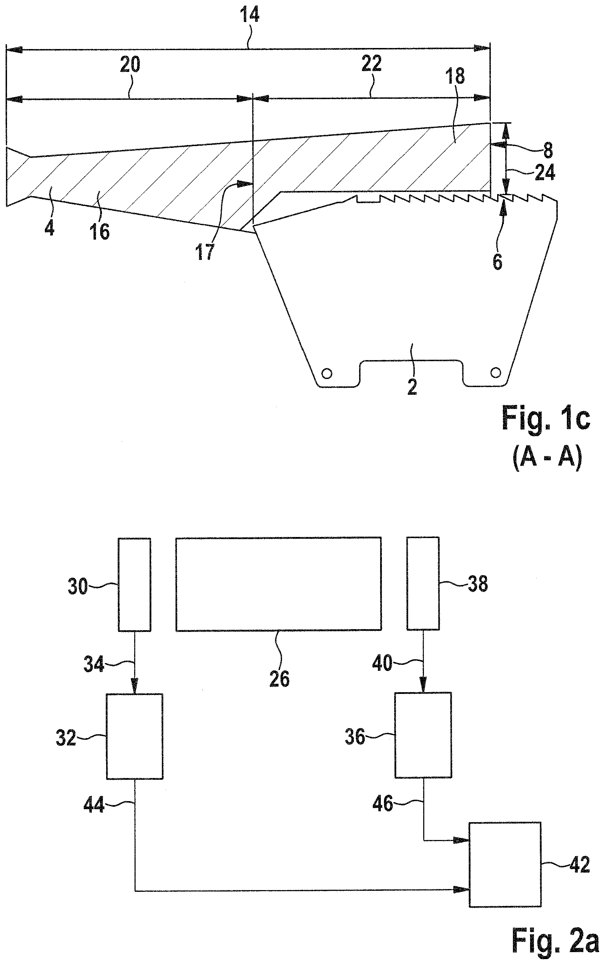

Zum besseren Verständnis der Erfindung wird zunächst in Fig. 1 ist ein Transportsattel 2 und ein Modell eines Fischrumpfes 4 dargestellt. Der Fischrumpf 4 ist dabei auf dem Transportsattel 2 befestigt. Der Transportsattel 2 weist dazu an dessen Oberkante Transportzähne 6 auf. Mittels des Transportsattels 2 wird der Fischrumpf 4 einer Fleischverarbeitungsvorrichtung zugeführt, innerhalb dieser befördert und von der Fischverarbeitungsvorrichtung abtransportiert. An dessen Schnittkante 8 hat der Fischrumpf 4 eine Produktbreite 10. Die Produktbreite 10 des Fischrumpfes 4 kann durch die Eingangssensoren einer Überwachungsvorrichtung erfasst werden.

In Fig. 1B ist die Vorderansicht der Schnittkante 8 des Fischrumpfes 4 dargestellt, wobei der Fischrumpf 4 auf dem Transportsattel 2 befestigt ist. Der Fischrumpf 4 hat an der Schnittkante 8 eine bestimmte Höhe 12. Des Weiteren ist in Fig. 1B der Schnitt A-A dargestellt.

Entsprechend des Schnittes A-A ist in Fig. 1C die Ansicht der Schnittebene A-A dargestellt. Gezeigt sind der Transportsattel 2 sowie die seitliche Schnittansicht des Fischrumpfes 4. Der Fischrumpf hat dabei eine Gesamtlänge 14. Des Weiteren sind in Fig. 1 C die Abschnitte 16 und 18 des Fischrumpfes 4 dargestellt, in die der Fischrumpf 4 durch die Fischverarbeitungsvorrichtung unterteilt werden soll. Der Abschnitt 16 des Fischrumpfes 4 hat dabei eine Länge 20, die kürzer ist als die Gesamtlänge 14 des Fischrumpfes 4. Entsprechend hat der andere Abschnitt 18 des Fischrumpfes 4 eine Länge 22, die auch kürzer ist als die Gesamtlänge 14 des Fischrumpfes 4. Der Fischrumpf 4 ist auf dem Transportsattel 2 in der Weise befestigt, dass die Schnittkante 8 um eine Distanz 24 über die Transportzähne 6 des Transportsattels 2 hinausragt.

Der Fischrumpf 4 kann die Basis für ein Morphologiemodell sein. Bei einem Außendurchmesser ($D_1$) der vorderen Schnittkante 8, einem Außendurchmesser ($D_2$) an der anderen Abschnittsgrenze 17 und einer Abschnittlänge (L) 22, weist Abschnitt ein Volumen von

$$V = \frac{L * \Pi}{3} * \left( \left( \frac{D_1}{2} \right)^2 + \left( \frac{D_1 * D_2}{4} \right) + \left( \frac{D_2}{2} \right)^2 \right)$$

auf. Das Gewicht ermittelt sich aus dem Produkt der spezifischen Dichte des Fisches und dem Volumen.

In Fig. 2A ist ein Blockschaltbild einer Überwachungsvorrichtung zur automatischen Überwachung einer Vorrichtung zur Verarbeitung von Fleischprodukten, insbesondere Fisch, dargestellt. Die Vorrichtung zur Verarbeitung von Fleischprodukten, insbesondere Fisch, kann auch als Fleischverarbeitungsvorrichtung 26 bezeichnet sein. Der mindestens eine Eingangssensor 30 dient zur Erfassung von Geometrie- und/oder Gewichtsdaten des der Fleischverarbeitungsvorrichtung 26 zugeführten Eingangsfleischproduktes. Dabei wird das Fleischprodukt von den Eingangssensoren 30 vorzugsweise auf einem Transportsattel erfasst. Zur Auswertung der Geometrie- und/oder Gewichtsdaten ist eine Prädiktionseinheit 32 mittels einer Datenverbindung 34 mit dem mindestens einen Eingangssensor 30 verbunden. Eine Prädiktionseinheit 32 kann grundsätzlich, insbesondere ausschließlich, zur Ermittlung einer ausbeuterelevanten Prädiktionsgröße eingerichtet und/oder ausgestaltet sein.

Grundsätzlich kann eine Datenverbindung 34 zwischen der Prädiktionseinheit 32 und dem mindestens einen Eingangssensor 30 eine beliebige Art einer Datenverbindung sein. Dies gilt im Übrigen auch für die nun folgenden genannten Datenverbindungen. Eine Datenverbindung kann insbesondere eine leitungsgebundene, eine Funk- und/oder eine Netzwerk-Verbindung sein.

Des Weiteren ist in Fig. 2A eine Ertragsermittlungseinheit 36 zur Ermittlung mindestens einer ausbeuterelevanten Ertragsgröße dargestellt. Die Ertragsermittlungseinheit 36 ist mit dem mindestens einen Ausgangssensor 38 zur Erfassung von Geometrie- und/oder Gewichtsdaten des von der Fleischverarbeitungseinrichtung 26 verarbeiteten Fleischproduktes, insbesondere des Ausbeutefleischproduktes, mittels einer weiteren Datenverbindung 40 verbunden. Die Ertragsermittlungseinheit 36 kann insbesondere ausschließlich dazu ausgestaltet und/oder eingerichtet sein, um ausbeuterelevante Ertragsgrößen zu ermitteln.

Des Weiteren ist in Fig. 2A eine Differenzeinheit 42 zur Berechnung einer Differenzgröße aus der Differenz einer der Prädiktionsgrößen und der mindestens einen entsprechenden Ertragsgröße dargestellt. Zur Übermittlung der von der Ertragsermittlungseinheit 36 ermittelten Ertragsgröße an die Differenzeinheit 42 ist die Differenzeinheit 42 mit der Ertragsermittlungseinheit 36 durch eine weitere Datenverbindung 46 verbunden. Mittels der Datenverbindung 46 zwischen der Differenzeinheit 42 und der Ertragsermittlungseinheit 36 kann die Differenzeinheit 42 zur Berechnung auf die jeweilige Ertragsgröße zugreifen. Des Weiteren ist die Differenzeinheit 42 mit der Prädiktionseinheit 32 durch eine weitere Datenverbindung 44 verbunden. Durch diese Verbindung kann die durch die Prädiktionseinheit 32 ermittelte Prädiktionsgröße an die Differenzeinheit 42 übermittelt werden. Die Differenzeinheit 42 kann also zur Berechnung der Differenzgröße mittels der Datenverbindung 44 auf die Prädiktionsgröße zugreifen.

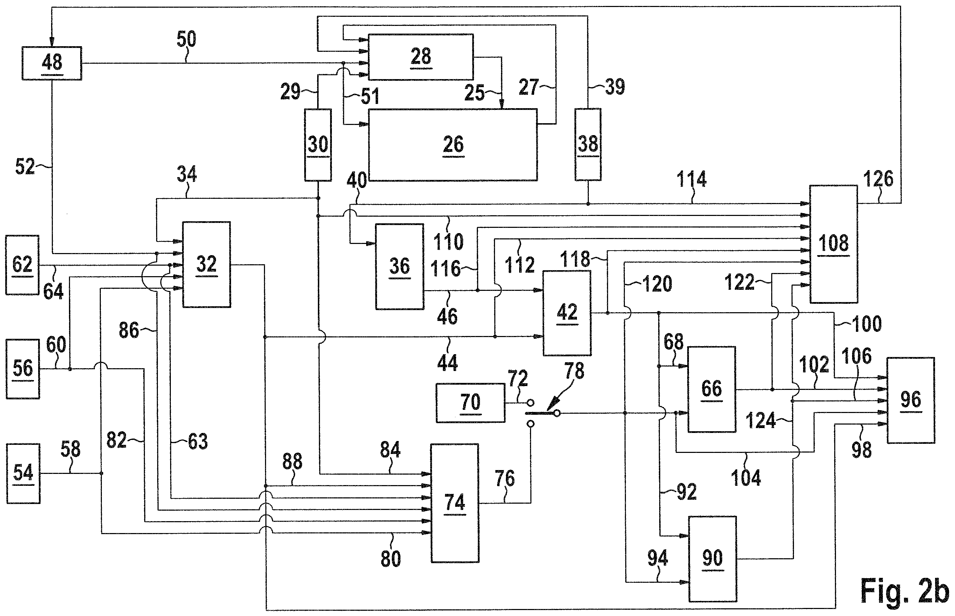

Weitere vorteilhafte Ausgestaltungen und Einzelheiten der Erfindung sind in der Figur 2B dargestellt. Dabei werden auf die Ausführungen zu Fig. 2A Bezug genommen, denn Fig. 2A bildet die Basis für die Fig. 2B. So sind auch in Fig. 2B die Fleischverarbeitungsvorrichtung 26, der Eingangssensor 30, der Ausgangssensor 38, die Prädiktionseinheit 32, die Betragsermittlungseinheit 36, die Differenzeinheit 42 sowie die Datenverbindungen 34, 40, 44 und 46 dargestellt. Die strukturellen und/oder funktionellen Zusammenhänge zwischen der Vorrichtung, den Sensoren, den Einheiten und den Datenverbindung entsprechen dabei den zu Fig. 2A erläuterten Zusammenhängen.

Zum besseren Verständnis der Erfindung ist neben der Fleischverarbeitungsvorrichtung 26 auch ein Steuerungs- und Regelungseinheit 28 dargestellt. Die Fleischverarbeitungsvorrichtung 26 kann mit der Steuerungs- und Regelungseinheit 28 verbunden sein, um die Fleischverarbeitungsvorrichtung 26 zu steuern beziehungsweise zur regeln. Dazu kann jeweils der Eingangssensor 30 mittels einer Datenverbindung 29, der Ausgangssensor 38 mittels einer Datenverbindung 39 sowie weitere (nicht dargestellte) Sensoren der Fleischverarbeitungsvorrichtung 26 mittels einer Datenverbindung 27 mit der Steuerungs- und Regelungseinheit 28 verbunden sein. Außerdem kann die Steuerungs- und Regelungseinheit 28 mittels einer weiter Datenverbindung 25 zur Übertragung von Steuer- und/oder Regelungssignalen mit der Fleischverarbeitungsvorrichtung 26 verbunden sein.

Eine vorteilhafte Ausgestaltung der Erfindung zeichnet sich dadurch aus, dass die Eingangssensoren 30 die Länge, die Höhe, die Breite, den Durchmesser, das Volumen und/oder das Gewicht des zugeführten Eingangsfleischproduktes, insbesondere kontaktlos, messen. Dazu können die Eingangssensoren 30 mechanisch, induktiv, kapazitiv, optisch, mittels Ultraschall, mittels Radar und/oder durch Winkelbestimmung messen. Das Messen kann auch an dem bewegten Fleischprodukt erfolgen. Eine besonders einfache Ausgestaltung der Eingangssensoren 30 zeichnet sich dadurch aus, dass eine Lichtschrankenanordnung, mit mehreren Lichtschranken, quer zur Zuführungsrichtung des zuzuführenden Fleischproduktes angeordnet ist. Damit kann die Länge, die Höhe und/oder die Breite des zugeführten Eingangsfleischproduktes ermittelt werden, wobei jeweils die Zeit des durch das zugeführte Eingangsfleischprodukt durchbrochenen Lichtstrahls ausgewertet werden kann.

Eine weitere vorteilhafte Ausgestaltung der Erfindung zeichnet sich dadurch aus, dass die Ausgangssensoren 38 die Länge, die Höhe, die Breite, den Durchmesser, das Volumen und/oder das Gewicht des Ausbeutefleischproduktes, insbesondere kontaktbehaftet, messen. Das Messen der Ausgangssensoren 38 kann mechanisch, induktiv, kapazitiv, piezoelektrisch, optisch, mittels Ultraschall, mittels Radar, mittels DMS und/oder durch Winkelbestimmung erfolgen. Eine besonders einfache Ausgestaltung zeichnet sich dadurch aus, dass das Gewicht des Ausbeutefleischproduktes durch eine Abtransportvorrichtung gemessen wird. Die Abtransportvorrichtung kann eine Gewichtsmesseinheit aufweisen, die beispielsweise mittels DMS, induktiv und/oder kapazitiv das Gewicht des verarbeiteten und von der Abtransportiervorrichtung transportierten Fleischproduktes misst.

Die Einstellungen der Fleischverarbeitungsvorrichtung 26 können durch Maschinenparameter bestimmt sein. Diese Maschinenparameter können in einem Maschinenparameterspeicher 48 gespeichert sein. Um die Fleischverarbeitungsvorrichtung 26 zu steuern und/oder zu regeln, kann die Steuerungs- und/oder Regelungseinheit 28 mittels einer weiteren Datenverbindung 50 mit dem Maschinenparameterspeicher 48 verbunden sein. Außerdem kann die Fleischbearbeitungsvorrichtung 26 mittels einer Datenverbindung 51 mit dem Maschinenparameterspeicher 48 verbunden sein.

Des Weiteren kann die Überwachungsvorrichtung in der Weise ausgestaltet sein, dass der Maschinenparameterspeicher 48 mittels einer weiteren Datenverbindung 52 mit der Prädiktionseinheit 32 verbunden ist. Mittels dieser Datenverbindung können Maschinenparameter von dem Maschinenparameterspeicher 48 an die Prädiktionseinheit 32 übermittelt werden. Mit anderen Worten kann die Prädiktionseinheit 32 auf die Maschinenparameter des Maschinenparameterspeichers 48 mittels der Datenverbindung 52 zugreifen. Die Prädiktionseinheit 32 kann auch in der Weise ausgestaltet sein, dass sie ein Morphologiemodell und/oder ein Maschinenmodell aufweist. Es ist aber auch möglich, dass ein Morphologiemodellspeicher 54 mittels einer Datenverbindung 58 mit der Prädiktionseinheit 32 verbunden ist. Ferner ist es möglich, dass ein Maschinenmodellspeicher 56 mittels einer weiteren Datenverbindung 60 mit der Prädiktionseinheit 32 verbunden ist. Durch die jeweilige Datenverbindung kann die Prädiktionseinheit 32 auf das Morphologiemodell und/oder auf das Maschinenmodell zugreifen, um die Prädiktionsgröße aus den Eingangssignalen oder aus den Eingangssignalen und den Maschinenparametern zu berechnen.

Des Weiteren kann die Überwachungsvorrichtung eine Datenbank 62 aufweisen. In der Datenbank 62 können Prädiktionsgrößen in Abhängigkeit von Eingangssignalen oder Eingangssignalen und Maschinenparametern gespeichert sein. Mittels einer weiteren Datenverbindung 64 kann die Datenbank 62 mit der Prädiktionseinheit 32 verbunden sein. Durch die Datenverbindung 64 hat die Prädiktionseinheit 32 also Zugriff auf die Daten der Datenbank 62. Die Prädiktionseinheit 32 kann eingerichtet sein, um der Prädiktionsgröße in Abhängigkeit der Eingangssignale oder der Eingangssignale und der Maschinenparameter einen entsprechenden Wert aus der Datenbank 62 zuzuordnen. Auf die Eingangssignale hat die Prädiktionseinheit 32 Zugriff über die Datenverbindung 34 zwischen der Prädiktionseinheit 32 und dem mindestens einen Eingangssensor 30. Auf die Maschinenparameter hat die Prädiktionseinheit Zugriff durch die Datenverbindung 52 zwischen der Prädiktionseinheit 32 und dem Maschinenparameterspeicher 48.

Des Weiteren kann die Überwachungsvorrichtung eine Vergleichseinheit 66 aufweisen. Durch die Vergleichseinheit 66 kann eine Vergleichsgröße durch Vergleichen der Differenzgröße mit einer wahlweise vorbestimmten, berechneten oder zugeordneten Bezugsgröße ermittelt werden. Dazu ist die Vergleichseinheit 66 mittels einer weiteren Datenverbindung 68 mit der Differenzeinheit 42 verbunden. Mittels dieser Datenverbindung hat die Vergleichseinheit 66 Zugriff auf die Differenzgröße der Differenzeinheit 42. Eine vorbestimmte Bezugsgröße kann in einem Bezugsgrößenspeicher 70 gespeichert sein. Zwischen dem Bezugsgrößenspeicher 70 und der Vergleichseinheit 66 kann eine weitere Datenverbindung 72 ausgebildet sein.

Die Überwachungsvorrichtung kann auch eine Bezugsgrößenermittlungseinheit 74 aufweisen. Auch die Bezugsgrößenermittlungseinheit 74 kann mittels einer weiteren Datenverbindung 76 mit der Vergleichseinheit 66 verbunden sein. Ferner kann ein Schalter 78 vorgesehen sein, der die Vergleichseinheit 66 wahlweise mit dem Bezugsgrößenspeicher 70 oder der Bezugsgrößenermittlungseinheit 74 verbindet. Die Vergleichseinheit kann vorzugsweise ausschließlich dazu ausgestaltet und/oder eingerichtet sein, um eine Vergleichsgröße durch Vergleichen der Differenzgröße mit der Bezugsgröße zu ermitteln.

Die Bezugsgrößenermittlungseinheit 74 kann ein weiteres Maschinenmodell und/oder ein weiteres Morphologiemodell aufweisen. Das Maschinenmodell und/oder das Morphologiemodell ist hierbei vorzugsweise das gleiche Maschinenmodell beziehungsweise Morphologiemodell, wie es die Prädiktionseinheit 32 vorzugsweise aufweist. Es ist auch möglich, dass die Bezugsgrößenermittlungseinheit 74 mittels einer weiteren Datenverbindung 80 mit dem Morphologiemodellspeicher 54 verbunden ist. Mittels der Datenverbindung 80 hat die Bezugsgrößenermittlungseinheit 74 Zugriff auf das Morphologiemodell. Des Weiteren kann die Bezugsgrößenermittlungseinheit 74 mittels einer weiteren Datenverbindung 82 mit dem Maschinenmodellspeicher 56 verbunden sein. Mittels dieser Datenverbindung 82 hat die Bezugsgrößenermittlungseinheit 74 Zugriff auf das Maschinenmodell.

Die Bezugsgrößenermittlungseinheit 74 ist, insbesondere ausschließlich, zur Berechnung der Bezugsgröße aus den Eingangssignalen des mindestens einen Eingangssensors 30 und den Maschinenparameters mittels des Maschinenmodells und/oder des Morphologiemodells eingerichtet und/oder ausgestaltet. Der mindestens eine Eingangssensor 30 kann durch eine weitere Datenverbindung 84 mit der Bezugsgrößenermittlungseinheit 74 verbunden sein. Durch diese Datenverbindung kann die Bezugsgrößenermittlungseinheit 74 auf die Eingangssignale des mindestens einen Eingangssensors 30 zugreifen. Auch der Maschinenparameterspeicher 48 kann durch eine weitere Datenverbindung 86 mit der Bezugsgrößenermittlungseinheit 74 verbunden sein. Durch diese Datenverbindung kann die Bezugsgrößenermittlungseinheit 74 auf die Maschinenparameter zugreifen. In einer weiteren Ausgestaltung kann die Bezugsgrößenermittlungseinheit 74 zur Berechnung der Bezugsgröße aus der Prädiktionsgröße und den Maschinenparametern mittels des Maschinenmodells eingerichtet und/oder ausgestaltet sein. Die Bezugsgrößenermittlungseinheit 74 kann durch eine weitere Datenverbindung 88 mit der Prädiktionseinheit 32 verbunden sein. Durch diese Datenverbindung kann die Bezugsgrößenermittlungseinheit 74 auf die Prädiktionsgröße der Prädiktionseinheit 32 zugreifen. Die Bezugsgrößenermittlungseinheit 74 kann, insbesondere ausschließlich, zur Berechnung der Bezugsgröße aus den Eingangssignalen beziehungsweise der Prädiktionsgröße und den Maschinenparametern eingerichtet und/oder ausgestaltet sein.

Die Bezugsgrößenermittlungseinheit 74 kann eingerichtet und/oder ausgestaltet sein, um der Bezugsgröße in Abhängigkeit der Eingangssignale, der Prädiktionsgröße und/oder der Maschinenparameter ein entsprechenden Wert aus einer, insbesondere weiteren, Datenbank zuzuordnen. Bei der Datenbank kann es sich um die bereits zuvor genannte Datenbank 62 handeln. Dazu kann Bezugsgrößenermittlungseinheit 74 mittels einer weiteren Datenverbindung 63 mit der Datenbank 62 verbunden sein. In der Datenbank, insbesondere in der Datenbank 62, sind Bezugsgrößen in Abhängigkeit von Eingangssignalen, Prädiktionsgrößen und/oder Maschinenparameters gespeichert. Mit der Bezugsgrößenermittlungseinheit 74 können jeweils der mindestens eine Eingangssensor 30 durch die Datenverbindung 84, die Prädiktionseinheit 32 durch die Datenverbindung 88 und/oder der Maschinenparameterspeicher durch die Datenverbindung 86 verbunden sein. Die Bezugsgrößenermittlungseinheit 74 hat somit Zugriff auf die entsprechenden Signale beziehungsweise Größen der Prädiktionseinheit 32, des mindestens einen Eingangssensors 30, der Datenbank 62 und/oder des Maschinenparameterspeichers 48.

Die Überwachungsvorrichtung kann eine Steuersignalermittlungseinheit 90 zur Erzeugung eines Steuersignals aufweisen. Die Steuersignalermittlungseinheit 90 kann vorzugsweise ausschließlich zur Erzeugung eines Steuersignals eingerichtet und/oder ausgestaltet sein. Des Weiteren kann die Steuersignalermittlungseinheit 90 in der Weise eingerichtet und/oder ausgestaltet sein, dass ein Steuersignal erzeugt wird, wenn die Differenzgröße eine Toleranzgrenze der Bezugsgröße erreicht, überschreitet oder unterschreitet. Die Toleranzgröße kann dabei eine vorbestimmte Toleranzgröße sein. Die Toleranzgröße kann auch eine von der Bezugsgröße abhängige Größe sein. So kann die obere Toleranzgrenze beispielsweise 5% größer als die Bezugsgröße sein. Die untere Toleranzgrenze kann beispielsweise 5% kleiner als die Bezugsgröße sein. Somit ergäbe dies ein Toleranzbereich von 10% um die Bezugsgröße. Alternative Grenzen und/oder Bereiche für die Toleranz sind auch möglich. Die Toleranz, insbesondere deren Grenzen und/oder Bereiche, kann in einem Toleranzspeicher gespeichert sein. Ferner kann die Toleranz auch von außen vorgebbar und/oder bestimmt werden. Die Signalermittlungseinheit 90 kann zur Differenzbildung eingerichtet und/oder ausgestaltet sein. Ferner kann die Steuersignalermittlungseinheit 90 zum Vergleichen der Differenzgröße mit mindestens einer der Toleranzgrenzen ausgestaltet und/oder eingerichtet sein. Die Steuersignalermittlungseinheit 90 ist durch eine weitere Datenverbindung 92 mit der Differenzeinheit 42 verbunden. Durch diese Datenverbindung kann die Differenzgröße an die Steuersignalermittlungseinheit 90 übermittelt werden. Mit anderen Worten hat die Steuersignalermittlungseinheit 90 Zugriff auf die Differenzgrößen der Differenzeinheit 42. Die Steuersignalermittlungseinheit 90 kann durch eine weitere Datenverbindung 94 mit dem Bezugsgrößenspeicher 70 oder der Bezugsgrößenermittlungseinheit 74 verbunden sein. Die Steuersignalermittlungseinheit hat somit Zugriff auf die Bezugsgröße.

Die Überwachungsvorrichtung kann auch eine Ausgabeeinheit 96 zur akustischen und/oder optischen Ausgabe der Prädiktionsgröße, der Differenzgröße, der Bezugsgröße, der Vergleichsgröße und/oder des Steuersignals aufweisen. Mit anderen Worten kann sich eine vorteilhafte Ausgestaltung der Erfindung dadurch auszeichnen, dass die Prädiktionsgröße, die Differenzgröße, die Vergleichsgröße und/oder das Steuersignal optisch und/oder akustisch ausgegeben werden. Dazu kann die Ausgabeeinheit 96 vorgesehen sein. Die Ausgabeeinheit 96 kann einen Lautsprecher aufweisen. Die Ausgabeeinheit 96 kann einen Bildschirm und/oder Leuchtmittel aufweisen. Die Prädiktionseinheit 32 kann durch eine weitere Datenverbindung 98 mit der Ausgabeeinheit 96 verbunden sein. Die Differenzeinheit 42 kann durch eine Datenverbindung 100 mit der Ausgabevorrichtung 96 verbunden sein. Die Vergleichseinheit 66 kann durch eine weitere Datenverbindung 102 mit der Ausgabeeinrichtung 96 verbunden sein. Die Bezugsgrößenermittlungseinheit 74 kann durch eine weitere Datenverbindung 104 mit der Ausgabeeinheit 96 verbunden sein. Die Steuersignalermittlungseinheit 90 kann durch eine weitere Datenverbindung 106 mit der Ausgabeeinheit 96 verbunden sein. Durch diese Datenverbindungen hat die Ausgabeeinheit 96 auf die entsprechenden Größen beziehungsweise Signale der jeweiligen Einheit Zugriff.

Eine weitere vorteilhafte Ausgestaltung der Erfindung zeichnet sich durch ein Ermitteln von mindestens einem Maschinensollparameter mittels der Eingangssignale, der Prädiktionsgröße, der Ausgangssignale, der Ertragsgröße, der Differenzgröße, der Vergleichsgröße und/oder des Steuersignals aus. Grundsätzlich kann eine Vorrichtung zur Fleischverarbeitung 26 mehrere Maschinenparameter aufweisen. Durch die Maschinenparameter können beispielsweise die Messerabstände einer Fischverarbeitungsvorrichtung bestimmt sein. Ferner können durch die Eingangssignale und/oder durch die Prädiktionsgröße auf Geometrie- und/oder Gewichtsdaten des zu verarbeitenden Fisches und/oder Fleischproduktes geschlossen werden. Soll beispielsweise ein kurzer und breiter Fisch durch eine Fischverarbeitungsvorrichtung 26 verarbeitet werden, kann es notwendig sein, die Messerabstände eines Bauchmessers zu vergrößern. Dazu ist es notwendig, dass der entsprechende Parameter, der dem Abstand der Bauchmesser zugeordnet ist, entsprechend verändert, insbesondere vergrößert, wird. Somit kann beispielsweise aus den Eingangssignalen und/oder Prädiktionssignalen der Sollparameter für den Messerabstand des Bauchmessers ermittelt werden.

Insbesondere wenn Fleischprodukte oder Fische mit gleicher und/oder ähnlicher Außenkontur verarbeitet werden sollen, kann es sinnvoll sein, gemessene und/oder ermittelte Informationen durch die bereits verarbeiteten Fleischprodukte und/oder Fische in der Weise zu nutzen, um die Verarbeitung der noch zu verarbeitenden Fleischprodukte und/oder Fische zu verbessern. Es kann also sinnvoll sein, nicht nur mittels der Eingangssignale oder der Prädiktionsgröße einen Maschinensollparameter zu ermitteln. Es kann auch sinnvoll sein, mittels der Ausgangssignale, der Ertragsgröße, der Differenzgröße, der Vergleichsgröße und/oder des Steuersignals einen Maschinensollparameter zu ermitteln. Dies kann insbesondere für die Differenzgröße von Vorteil sein. So könnte ein Maschinensollparameter in der Weise ermittelt und/oder berechnet werden, indem zu dem Maschinenparameter eine von der Differenzgröße linear abhängige Größe addiert wird. In einem ganz besonders einfachen Fall wird der Maschinensollparameter berechnet, indem die Differenzgröße zu dem entsprechenden Maschinenparameter addiert wird. Entsprechende Ermittlungsverfahren und/oder Vorrichtungen können auch für die Ausgangssignale, die Ertragsgröße, die Vergleichsgröße und/oder das Steuersignal gelten beziehungsweise vorgesehen sein.

Die Überwachungsvorrichtung kann eine Parameterermittlungseinheit 108 zur Ermittlung von mindestens einem Maschinensollparameter mittels der Eingangssignale, der Prädiktionsgröße, der Ausgangssignale, der Ertragsgröße, der Differenzgröße, der Bezugsgröße, der Vergleichsgröße und/oder des Steuersignals aufweisen. Die Parameterermittlungseinheit 108 kann vorzugsweise ausschließlich zur Ermittlung von mindestens einem Maschinensollparameter ausgestaltet und/oder eingerichtet sein. Der mindestens eine Eingangssensor 30 kann durch eine weitere Datenverbindung 110 mit der Parameterermittlungseinheit 108 verbunden sein. Die Prädiktionseinheit 32 kann durch eine weitere Datenverbindung 112 mit der Parameterermittlungseinheit 108 verbunden sein. Der mindestens eine Ausgangssensor 38 kann durch eine weitere Datenverbindung 114 mit der Parameterverbindungseinheit 108 verbunden sein. Die Ertragsgrößenermittlungseinheit 36 kann durch eine weitere Datenverbindung 116 mit der Parameterermittlungseinheit 108 verbunden sein. Die Differenzeinheit 42 kann durch eine weitere Datenverbindung 118 mit der Parameterermittlungseinheit 108 verbunden sein. Die Bezugsgrößenermittlungseinheit 74 kann durch eine weitere Datenverbindung 120 mit der Parameterermittlungseinheit 108 verbunden sein. Die Vergleichseinheit 66 kann durch eine weitere Datenverbindung 122 mit der Parameterermittlungseinheit 108 verbunden sein. Die Steuersignalermittlungseinheit 90 kann durch eine weitere Datenverbindung 124 mit der Parameterermittlungseinheit 108 verbunden sein. Durch die Datenverbindungen mit der Parameterermittlungseinheit 108 hat diese Zugriff auf die entsprechenden Größen und/oder Signale der mit ihr verbundenen Einheiten beziehungsweise Sensoren.

Eine weitere vorteilhafte Ausgestaltung der Erfindung zeichnet sich durch ein Ersetzen von mindestens einem Maschinenparameter durch den entsprechenden mindestens einen Maschinensollparameter aus. Wird beispielsweise für den Messerabstand des Bauchmessers ein Maschinensollparameter ermittelt, so kann dieser vor der Verarbeitung des entsprechenden zu verarbeitenden Fisches anstatt des entsprechenden Maschinenparameters in einem Maschinenparameterspeicher 48 gespeichert werden. Mit anderen Worten kann der Maschinensollparameter den entsprechenden Maschinenparameter ersetzen. Es können auch mehrere Maschinensollparameter mittels der zuvor genannten Signale oder Größen ermittelt werden, die jeweils die entsprechenden Maschinenparameter ersetzen beziehungsweise anstatt der entsprechenden Parameter in einer Maschinenparameterspeicher 48 gespeichert werden.

Die Parameterermittlungseinheit 108 kann zum Ersetzen von mindestens einem Maschinenparameter aus dem Maschinenparameterspeicher 48 durch den entsprechenden mindestens einen Maschinensollparameter eingerichtet und/oder ausgestaltet sein. Der Maschinenparameterspeicher 48 kann mit der Parameterermittlungseinheit 108 durch eine weitere Datenverbindung 126 verbunden sein. Mittels dieser Datenverbindung hat die Parameterermittlungseinheit Zugriff auf den Maschinenparameterspeicher 48.

Eine weitere vorteilhafte Ausgestaltung der Erfindung zeichnet sich durch eine Aktualisierung mindestens einer der Datenbanken 62 durch die Speicherung von der Datenbank 62 zugehörigen Größen, insbesondere Eingangssignalen, Prädiktionsgrößen, Ausgangssignalen, Ertragsgrößen, Differenzgrößen, Bezugsgrößen, Vergleichgrößen, Steuersignalen und/oder Maschinenparametern aus. Werden beispielsweise bei der Verarbeitung von Fisch von einem zugeführten Fisch die Eingangssignale gemessen, daraus Prädiktionsgrößen ermittelt und nach dessen Verarbeitung Ausgangssignale gemessen und daraus wiederum Ertragsgrößen ermittelt, aus den entsprechenden Prädiktionsgrößen und Ertragsgrößen Differenzgrößen ermittelt, die jeweils zu aus den Prädiktionsgrößen und/oder Ertragsgrößen ermittelten Bezugsgrößen gesetzt werden, um daraus gegebenenfalls Vergleichsgrößen und/oder Steuersignale zu ermitteln, wobei die Vorrichtung den Fisch entsprechend der Einstellungen nach den Maschinenparametern verarbeitet hat, bildet dies beispielsweise einen Datensatz von zusammengehörigen Größen. Für jeden zu verarbeitenden Fisch werden zusammengehörige Größen ermittelt. Somit kann eine Vielzahl von Datensätzen bestehen. Diese können nach und nach in einer Datenbank 62 gespeichert werden. Werden Datensätze ermittelt, die bis auf eine oder mehrere Größen des Datensatzes eines in der Datenbank 62 gespeicherten Datensatzes entspricht, so kann beispielsweise in Abhängigkeit der Differenzgröße und/oder Vergleichsgröße und/oder Steuersignal der ermittelte, insbesondere neue, Datensatz den bereits in der Datenbank 62 gespeicherten Datensatz ersetzen. Anderenfalls kann der bereits gespeicherte Datensatz in der Datenbank 62 verbleiben. Auch ist es möglich, dass sowohl der bereits gespeicherte Datensatz als auch der neue Datensatz in der Datenbank 62 gespeichert wird. Des Weiteren ist es möglich, dass die zuvor genannten Datenbanken in einer gemeinsamen Datenbank 62 integriert sind. Somit kann es sich bei den Datenbanken um eine einzige Datenbank handeln.

Die Parameterermittlungseinheit 108 kann die Maschinenparameter mit mindestens einem Maschinensollparameter aktualisieren. Insbesondere ist die Parameterermittlungseinheit dazu eingerichtet und/oder ausgestaltet, denjenigen Maschinenparameter aus dem Maschinenparameterspeicher 48 durch den Maschinensollparameter zu ersetzen, der mit dem Maschinensollparameter die höchste Ähnlichkeit und/oder die kleinste Differenz aufweist. Alternativ kann der Maschinensollparameter in dem Maschinenparameterspeicher 48 zusätzlich gespeichert werden. Unter dem Maschinenparameter kann insbesondere ein Maschinenparametersatz mit mehren einzelnen Parametern verstanden werden.

## Patentansprüche

1. Verfahren zur automatischen Überwachung einer Vorrichtung **(26)** zur Verarbeitung von Fleischprodukten, insbesondere Fisch, **gekennzeichnet durch** die folgenden Schritte:

   a. Messen von Eingangssignalen von Eingangssensoren **(30)** zur Erfassung von Geometrie- und/oder Gewichtsdaten eines der Vorrichtung **(26)** zugeführten Eingangsfleischproduktes **(4)** und zur Ermittlung mindestens einer ausbeuterelevanten Prädiktionsgröße,
   b. Messen von Ausgangssignalen von Ausgangssensoren **(38)** zur Erfassung von Geometrie- und/oder Gewichtsdaten eines Ausbeutefleischproduktes und zur Ermittlung mindestens einer ausbeuterelevanten Ertragsgröße, und
   c. Berechnen einer Differenzgröße mittels einer Differenzeinheit **(42) durch** Differenzbildung zwischen einer der Prädiktionsgrößen und der mindestens einen entsprechenden Ertragsgröße.

2. Verfahren nach Anspruch 1, **dadurch gekennzeichnet, dass** die Prädiktionsgröße aus den Eingangssignalen mittels eines Morphologiemodells oder aus den Eingangssignalen und Maschinenparametern mittels eines Morphologiemodells und eines Maschinenmodells berechnet wird.

3. Verfahren nach Anspruch 1, **dadurch gekennzeichnet, dass** der Prädiktionsgröße in Abhängigkeit der Eingangssignale oder der Eingangssignale und Maschinenparameter ein entsprechender Wert aus einer Datenbank **(62)** zugeordnet wird, wobei in der Datenbank **(62)** Prädiktionsgrößen in Abhängigkeit von Eingangssignalen oder Eingangssignalen und Maschinenparametern gespeichert sind.

4. Verfahren nach einem der Ansprüche 1 bis 3, **gekennzeichnet durch** ein Ermitteln einer Vergleichsgröße **durch** Vergleichen der Differenzgröße mit einer wahlweise vorbestimmten, berechneten oder zugeordneten Bezugsgröße.

5. Verfahren nach einem der Ansprüche 1 bis 4, **gekennzeichnet durch** ein Berechnen der Bezugsgröße aus den Eingangssignalen und den Maschinenparametern mittels des Maschinenmodells und/oder des Morphologiemodells.

6. Verfahren nach einem der Ansprüche 1 bis 4, **dadurch gekennzeichnet, dass** der Bezugsgröße in Abhängigkeit der Eingangssignale und Maschinenparameter oder der Prädiktionsgröße und Maschinenparameter ein entsprechender Wert aus einer Datenbank **(62)** zugeordnet wird, wobei in der Datenbank **(62)** Bezugsgrößen in Abhängigkeit von Eingangssignalen und Maschinenparametern oder Prädiktionsgrößen und Maschinenparametern gespeichert sind.

7. Verfahren nach einem der Ansprüche 4 bis 6, **gekennzeichnet durch** ein Erzeugen eines Steuersignals, wenn die Differenzgröße eine Toleranzgrenze der Bezugsgröße erreicht, überschreitet oder unterschreitet.

8. Verfahren nach einem der Ansprüche 1 bis 7, **gekennzeichnet durch** ein Identifizieren eines Abschnittes **(16, 18)** des zugeführten Eingangsfleischproduktes **(4)** aus den Eingangssignalen mittels eines Morphologiemodells.

9. Verfahren nach einem der Ansprüche 1 bis 7, **dadurch gekennzeichnet, dass** ein Abschnitt **(16, 18)** des zugeführten Eingangsfleischproduktes **(4)** in Abhängigkeit der Eingangssignale mittels einer Datenbank **(62)** identifiziert wird, wobei in der Datenbank **(62)** Abschnittsdaten von Fleischprodukten in Abhängigkeit von Eingangssignalen gespeichert sind.

10. Überwachungsvorrichtung zur automatischen Überwachung einer Vorrichtung **(26)** zur Verarbeitung von Fleischprodukten, insbesondere Fisch,
**gekennzeichnet durch** eine Prädiktionseinheit **(32)** zur Ermittlung einer ausbeuterelevanten Prädiktionsgröße, wobei die Prädiktionseinheit **(32)** mit Eingangssensoren **(30)** zur Erfassung von Geometrie- und/oder Gewichtsdaten eines der Vorrichtung **(26)** zur Fleischverarbeitung zugeführten Eingangsfleischproduktes **(4)** mittels einer Datenverbindung **(34)** verbunden ist, eine Ertragsermittlungseinheit **(36)** zur Ermittlung mindestens einer ausbeuterelevanten Ertragsgröße, wobei die Ertragsermittlungseinheit **(36)** mit den Ausgangssensoren **(38)** zur Erfassung von Geometrie- und/oder Gewichtsdaten eines Ausbeutefleischproduktes mittels einer weiteren Datenverbindung **(40)** verbunden ist, und eine Differenzeinheit **(42)** zur Berechnung einer Differenzgröße aus der Differenz zwischen einer der Prädiktionsgrößen und der mindestens einen entsprechenden Ertragsgröße, wobei die Differenzeinheit **(42)** mit der Prädiktionseinheit **(32) durch** eine weitere Datenverbindung **(44)** verbunden ist, und wobei die Differenzeinheit **(42)** mit der Ertragsermittlungseinheit **(36) durch** eine weitere Datenverbindung **(46)** verbunden ist.

11. Überwachungsvorrichtung nach Anspruch 10, **dadurch gekennzeichnet, dass** die Prädiktionseinheit **(32)** ein Morphologiemodell zur Berechnung der Prädiktionsgröße aus den Eingangssignalen aufweist oder dass die Prädiktionseinheit **(32)** ein Morphologiemodell und ein Maschinenmodell zur Berechnung der Prädiktionsgröße aus den Eingangssignalen und den Maschinenparametern aufweist, wobei ein Maschinenparameterspeicher **(48)** zur Speicherung von Maschinenparametern mittels einer weiteren Datenverbindung **(52)** mit der Prädiktionseinheit **(32)** verbunden ist.

12. Überwachungsvorrichtung nach Anspruch 10, **dadurch gekennzeichnet, dass** eine Datenbank **(62),** in der Prädiktionsgrößen in Abhängigkeit von Eingangssignalen oder Eingangssignalen und Maschinenparametern gespeichert sind, mittels einer weiteren Datenverbindung **(64)** mit der Prädiktionseinheit **(32)** verbunden ist, und dass die Prädiktionseinheit **(32)** eingerichtet ist, um der Prädiktionsgröße in Abhängigkeit der Eingangssignale oder der Eingangssignale und Maschinenparameter einen entsprechenden Wert aus der Datenbank **(62)** zuzuordnen.

13. Überwachungsvorrichtung nach einem der Ansprüche 10 bis 12, **gekennzeichnet durch** eine Vergleichseinheit **(66)** zum Ermitteln einer Vergleichsgröße **durch** Vergleichen der Differenzgröße mit einer wahlweise vorbestimmten, berechneten oder zugeordneten Bezugsgröße, wobei die Vergleichseinheit **(66) durch** eine weitere Datenverbindung **(68)** mit der Differenzeinheit **(42)** verbunden ist.

14. Überwachungsvorrichtung nach Anspruch 13, **gekennzeichnet durch** eine Bezugsgrößenermittlungseinheit **(74)** mit einem weiteren Maschinenmodell und/oder Morphologiemodell zur Berechnung der Bezugsgröße aus den Eingangssignalen und den Maschinenparametern oder aus der Prädiktionsgröße und den Maschinenparametern mittels des Maschinenmodells und/oder des Morphologiemodells, wobei die Eingangssensoren **(30),** der Maschinenparameterspeicher **(48)** und die Vergleichseinheit **(66)** jeweils **durch** eine weitere Datenverbindung **(84, 86, 76)** mit der Bezugsgrößenermittlungseinheit **(74)** verbunden sind.

15. Überwachungsvorrichtung nach Anspruch 13, **dadurch gekennzeichnet, dass** eine Bezugsgrößenermittlungseinheit **(74)** eingerichtet ist, um der Bezugsgröße in Abhängigkeit der Eingangssignale, der Prädiktionsgröße und/oder der Maschinenparameter einen entsprechenden Wert aus einer Datenbank **(62)** zuzuordnen, wobei in der Datenbank **(62)** Bezugsgrößen in Abhängigkeit von Eingangssignalen, Prädiktionsgrößen und/oder Maschinenparametern gespeichert sind, und wobei die Eingangssensoren **(30),** die Prädiktionseinheit **(32)** und/oder der Maschinenparameterspeicher **(48)** jeweils mittels einer weiteren Datenverbindung **(84, 88, 86)** mit der Bezugsgrößenermittlungseinheit verbunden sind.

16. Überwachungsvorrichtung nach einem der Ansprüche 13 bis 15, **gekennzeichnet durch** eine Steuersignalermittlungseinheit **(90)** zur Erzeugung eines Steuersignals, wenn die Differenzgröße eine Toleranzgrenze der Bezugsgröße erreicht, überschreitet oder unterschreitet, wobei die Differenzeinheit **(42)** oder die Differenzeinheit **(42)** und die Bezugsgrößenermittlungseinheit **(74)** jeweils mittels einer weiteren Datenverbindung **(92, 94)** mit der Steuersignalermittlungseinheit **(90)** verbunden sind.


**Claims**

1. Method for automatically monitoring an apparatus **(26)** for processing meat products, in particular fish, **characterised by** the following steps:

   a. measuring input signals from input sensors **(30)** for acquiring geometric and/or weight data of an input meat product **(4)** fed to the apparatus **(26)** and for determining at least one yield-relevant prediction variable,
   b. measuring output signals from output sensors **(38)** for acquiring geometric and/or weight data of a meat product yield and for determining at least one yield-relevant yield variable, and
   c. calculating a difference variable by means of a difference unit **(42)** by calculating a difference between one of the prediction variables and the at least one corresponding yield variable.

2. Method according to claim 1, **characterised in that** the prediction variable is calculated from the input signals by means of a morphology model or from the input signals and machine parameters by means of a morphology model and a machine model.

3. Method according to claim 1, **characterised in that** a corresponding value from a database **(62)** is assigned to the prediction variable depending on the input signals or the input signals and machine parameters, prediction variables being stored in the database **(62)** depending on input signals or input signals and machine parameters.

4. Method according to any one of claims 1 to 3, **characterised by** determining a comparison variable by comparing the difference variable with a reference variable which is optionally predetermined, calculated or assigned.

5. Method according to any one of claims 1 to 4, **characterised by** calculating the reference variable from the input signals and the machine parameters by means of the machine model and/or the morphology model.

6. Method according to any one of claims 1 to 4, **characterised in that** a corresponding value from a database **(62)** is assigned to the reference variable depending on the input signals and machine parameters or the prediction variable and machine parameters, reference variables depending on input signals and machine parameters or prediction variables and machine parameters being stored in the database **(62)**.

7. Method according to any one of claims 4 to 6, **characterised by** generating a control signal when the difference variable reaches, exceeds or falls below a tolerance threshold of the reference variable.

8. Method according to any one of claims 1 to 7, **characterised by** the identification of a portion **(16, 18)** of the fed input meat product **(4)** from the input signals by means of a morphology model.

9. Method according to any one of claims 1 to 7, **characterised in that** a portion **(16, 18)** of the fed input meat product **(4)** is identified by means of a database **(62)** depending on the input signals, portion data of meat products depending on input signals being stored in the database **(62).**

10. Monitoring apparatus for automatically monitoring an apparatus **(26)** for processing meat products, in particular fish, **characterised by** a prediction unit **(32)** for determining a yield-relevant prediction variable, the prediction unit **(32)** being connected by means of a data connection **(34)** with input sensors **(30)** for acquiring geometric and/or weight data of an input meat product **(4)** fed to the apparatus **(26)** for meat processing, a yield determining unit **(36)** for determining at least one yield-relevant yield variable, the yield determining unit **(36)** being connected to the output sensors **(38)** for acquiring geometric and/or weight data of a meat product yield by means of a further data connection **(40),** and a difference unit **(42)** for calculating a difference variable from the difference between one of the prediction variables and the at least one corresponding yield variable, the difference unit **(42)** being connected to the prediction unit **(32)** by a further data connection **(44)** and the difference unit **(42)** being connected to the yield determining unit **(36)** by a further data connection **(46).**

11. Monitoring apparatus according to claim 10, **characterised in that** the prediction unit **(32)** has a morphology model for calculating the prediction variable from the input signals or the prediction unit **(32)** has a morphology model and a machine model for calculating the prediction variable from the input signals and the machine parameters, a machine parameter memory **(48)** being connected to the prediction unit **(32)** by means of a further data connection (52) for storing machine parameters.

12. Monitoring apparatus according to claim 10, **characterised in that** a database **(62)** in which prediction variables are stored depending on input signals or input signals and machine parameters, is connected to the prediction unit **(32)** by means of a further data connection **(64),** and the prediction unit **(32)** is designed to assign a corresponding value from the database **(62)** to the prediction variable depending on the input signals or the input signals and machine parameters.

13. Monitoring apparatus according to any one of claims 10 to 12, **characterised by** a comparison unit **(66)** for determining a comparison variable by comparing the difference variable with an optionally predetermined, calculated or assigned reference variable, the comparison unit **(66)** being connected by a further data connection **(68)** to the difference unit **(42).**

14. Monitoring apparatus according to claim 13, **characterised by** a reference variable determining unit **(74)** with a further machine model and/or morphology model for calculating the reference variable from the input signals and the machine parameters or from the prediction variable and the machine parameters by means of the machine model and/or the morphology model, the input sensors **(30),** the machine parameter memory **(48)** and the comparison unit **(66)** in each case being connected by a further data connection **(84, 86, 76)** to the reference variable determining unit **(74).**

15. Monitoring apparatus according to claim 13, **characterised in that** a reference variable determining unit **(74)** is designed to assign a corresponding value from a database **(62)** to the reference variable depending on the input signals, the prediction variable and/or the machine parameters, reference variables depending on input signals, prediction variables and/or machine parameters being stored in the database **(62),** and the input sensors **(30),** the prediction unit **(32)** and/or the machine parameter memory **(48)** in each case being connected by means of a further data connection **(84, 88, 86)** to the reference variable determining unit.

16. Monitoring apparatus according to any one of claims 13 to 15, **characterised by** a control signal determining unit **(90)** for producing a control signal when the difference variable reaches, exceeds or falls below a tolerance threshold of the reference variable, the difference unit **(42)** or the difference unit **(42)** and the reference variable determining unit **(74)** in each case being connected by means of a further data connection **(92, 94)** to the control signal determining unit **(90).**

**Revendications**

1. Procédé de contrôle automatique d'un dispositif **(26)** pour le traitement de produits à chair, notamment de poisson, **caractérisé par** les étapes suivantes :

a. mesure de signaux entrants émis par des capteurs d'entrée **(30),** afin d'obtenir des données géométriques et/ou relatives au poids d'un produit à chair entrant **(4)** introduit dans le dispositif **(26)** et afin de déterminer au moins une grandeur de prédiction pertinente en matière de résultat de production,

b. mesure de signaux sortants émis par des capteurs de sortie **(38),** afin d'obtenir des données géométriques et/ou relatives au poids d'un produit à chair de résultat de production et afin de déterminer au moins une grandeur de rendement pertinente en matière de résultat de production, et

c. calcul d'une grandeur différentielle au moyen d'une unité différentielle **(42)** par formation de différence entre l'une des grandeurs de prédiction et l'au moins une grandeur de rendement correspondante.

2.  Procédé selon la revendication 1, **caractérisé en ce que** la grandeur de prédiction est, à partir des signaux entrants, calculée au moyen d'un modèle morphologique ou, à partir des signaux entrants et paramètres machine, au moyen d'un modèle morphologique et d'un modèle machine.

3.  Procédé selon la revendication 1, **caractérisé en ce qu'**une valeur correspondante issue d'une banque de données **(62)** est associée à la grandeur de prédiction en fonction des signaux entrants ou des signaux entrants et paramètres machine, des grandeurs de prédiction étant enregistrées dans la banque de données **(62)** en fonction de signaux entrants ou de signaux entrants et paramètres machine.

4.  Procédé selon l'une quelconque des revendications 1 à 3, **caractérisé en ce qu'**une grandeur de comparaison est déterminée par comparaison de la grandeur différentielle à une grandeur de référence, au choix prédéterminée, calculée ou associée.

5.  Procédé selon l'une quelconque des revendications 1 à 4, **caractérisé en ce que** la grandeur de référence est calculée à partir des signaux entrants et des paramètres machine au moyen du modèle machine et/ou du modèle morphologique.

6.  Procédé selon l'une quelconque des revendications 1 à 4, **caractérisé en ce qu'**une valeur correspondante issue d'une banque de données **(62)** est associée à la grandeur de référence en fonction des signaux entrants et paramètres machine ou de la grandeur de prédiction et des paramètres machine, des grandeurs de référence étant enregistrées dans la banque de données **(62)** en fonction de signaux entrants et paramètres machine ou de grandeurs de prédiction et paramètres machine.

7.  Procédé selon l'une quelconque des revendications 4 à 6, **caractérisé en ce qu'**un signal de commande est généré lorsque la grandeur différentielle atteint, dépasse ou est inférieure à une limite de tolérance de la grandeur de référence.

8.  Procédé selon l'une quelconque des revendications 1 à 7, **caractérisé en ce qu'**une portion **(16, 18)** du produit à chair entrant **(4)** introduit est identifiée à partir des signaux entrants au moyen d'un modèle morphologique.

9.  Procédé selon l'une quelconque des revendications 1 à 7, **caractérisé en ce qu'**une portion **(16, 18)** du produit à chair entrant **(4)** introduit est identifiée en fonction des signaux entrants au moyen d'une banque de données **(62),** des données relatives aux portions de produits à chair étant enregistrées dans ladite banque de données **(62)** en fonction des signaux entrants.

10. Dispositif de contrôle destiné au contrôle automatique d'un dispositif **(26)** destiné au traitement de produits à chair, notamment de poisson, **caractérisé par** une unité de prédiction **(32)** destinée à la détermination d'une grandeur de prédiction pertinente en matière de résultat de production, ladite unité de prédiction **(32)** étant connectée à des capteurs d'entrée **(30)** par une connexion de données **(34)** afin d'obtenir des données géométriques et/ou relatives au poids d'un produit à chair entrant **(4)** introduit dans le dispositif **(26)** pour le traitement de la chair, en une unité de détermination de rendement **(36)** destinée à la détermination d'au moins une grandeur de rendement pertinente en matière de résultat de production, ladite unité de détermination de rendement **(36)** étant connectée par une autre connexion de données **(40)** aux capteurs de sortie **(38)** afin d'obtenir des données géométriques et/ou relatives au poids d'un produit à chair de résultat de production, et en une unité différentielle **(42)** destinée au calcul d'une grandeur différentielle à partir de la différence entre une des grandeurs de prédiction et l'au moins une grandeur de rendement correspondante, l'unité différentielle **(42)** étant connectée à l'unité de prédiction **(32)** par une autre connexion de données **(44),** et l'unité différentielle **(42)** étant connectée à l'unité de détermination de rendement **(36)** par une autre connexion de données **(46).**

**11.** Dispositif de contrôle selon la revendication 10, **caractérisé en ce que** l'unité de prédiction **(32)** présente un modèle morphologique destiné au calcul de la grandeur de prédiction à partir des signaux entrants ou que l'unité de prédiction **(32)** présente un modèle morphologique et un modèle machine destinés au calcul de la grandeur de prédiction à partir des signaux entrants et des paramètres machine, une mémoire pour paramètres machine **(48)** étant connectée au moyen d'une autre connexion de données **(52)** à l'unité de prédiction **(32)** pour enregistrer des paramètres machine.

**12.** Dispositif de contrôle selon la revendication 10, **caractérisé en ce qu'**une banque de données **(62)** dans laquelle des grandeurs de prédiction sont enregistrées en fonction de signaux entrants ou de signaux entrants et paramètres machine, est connectée au moyen d'une autre connexion de données **(64)** à l'unité de prédiction **(32),** et que l'unité de prédiction **(32)** est conçue pour associer à la grandeur de prédiction une valeur correspondante issue de la banque de données **(62)** en fonction des signaux entrants ou des signaux entrants et paramètres machine.

**13.** Dispositif de contrôle selon l'une quelconque des revendications 10 à 12, caractérisé en une unité de comparaison **(66)** destinée à déterminer une grandeur de comparaison par comparaison de la grandeur différentielle à une grandeur de référence, au choix prédéterminée, calculée ou associée, ladite unité de comparaison **(66)** étant connectée par une autre connexion de données **(68)** à l'unité différentielle **(42).**

**14.** Dispositif de contrôle selon la revendication 13, caractérisé en une unité de détermination de grandeurs de référence **(74)** dotée d'un autre modèle machine et/ou modèle morphologique destiné(s) au calcul de la grandeur de référence à partir des signaux entrants et des paramètres machine ou à partir de la grandeur de prédiction et des paramètres machine au moyen du modèle machine et/ou du modèle morphologique, les capteurs d'entrée **(30),** la mémoire pour paramètres machine **(48)** et l'unité de comparaison **(66)** étant chacun connectés par une autre connexion de données **(84, 86, 76)** à l'unité de détermination de grandeurs de référence **(74).**

**15.** Dispositif de contrôle selon la revendication 13, **caractérisé en ce qu'**une unité de détermination de grandeurs de référence **(74)** est conçue pour associer à la grandeur de référence, en fonction des signaux entrants, de la grandeur de prédiction et/ou des paramètres machine, une valeur correspondante issue d'une banque de données **(62),** des grandeurs de référence étant enregistrées dans la banque de données **(62)** en fonction de signaux entrants, grandeurs de prédiction et/ou paramètres machine, et les capteurs d'entrée **(30),** l'unité de prédiction **(32)** et/ou la mémoire pour paramètres machine **(48)** étant chacun connectés au moyen d'une autre connexion de données **(84, 88, 86)** à l'unité de détermination de grandeurs de référence.

**16.** Dispositif de contrôle selon l'une quelconque des revendications 13 à 15, caractérisé en une unité de détermination de signaux de commande **(90)** destinée à générer un signal de commande lorsque la grandeur différentielle atteint, dépasse ou est inférieure à une limite de tolérance de la grandeur de référence, l'unité différentielle **(42)** ou l'unité différentielle **(42)** et l'unité de détermination de grandeurs de référence **(74)** étant chacune connectées à l'unité de détermination de signaux de commande **(90)** au moyen d'une autre connexion de données **(92, 94).**

Fig. 1a

Fig. 1b

Fig. 1c
(A - A)

Fig. 2a

Fig. 2b

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4204843 A1

- US 7251537 B1